# EUROPÄISCHE PATENTSCHRIFT

(11) **EP 0 592 478 B1**
(45) Veröffentlichungstag und Bekanntmachung des Hinweises auf die Patenterteilung: **30.12.1998**
(21) Anmeldenummer: 92912628.2
(22) Anmeldetag: 23.06.1992
(51) Int. Cl.: A61K 38/46, C12N 9/20, C12N 1/20

(54) **VERWENDUNG VON LIPASEN ZUR HERSTELLUNG VON ARZNEIMITTELN**
USE OF LIPASES TO PRODUCE DRUGS
UTILISATION DE LIPASES POUR LA FABRICATION DE MEDICAMENTS

(30) Priorität: 01.07.1991 DE 4121704
(43) Veröffentlichungstag der Anmeldung: 20.04.1994
(73) Patentinhaber: BASF Aktiengesellschaft, 67063 Ludwigshafen (DE)
(72) Erfinder: BRAATZ, Reinhard, D-2000 Wedel (DE); KURTH, Roland, D-6703 Limburgerhof (DE); MENKEL-CONEN, Elke, D-6720 Speyer (DE); RETTENMAIER, Hansjoerg, D-6718 Gruenstadt (DE); FRIEDRICH, Thomas, D-6100 Darmstadt (DE); SUBKOWSKI, Thomas, D-6701 Mutterstadt (DE)
(86) Internationale Anmeldenummer: EP9201412
(87) Internationale Veröffentlichungsnummer: WO9300924

(56) Entgegenhaltungen:
- EP-A- 0 318 775
- EP-A- 0 331 376
- EP-A- 0 387 945
- DE-A- 1 642 654
- DE-A- 1 932 981
- DE-B- 1 300 487
- CHEMICAL ABSTRACTS, vol. 76, no. 13, 27. März 1972, Columbus, Ohio, US; abstract no. 70997V, 'Thermostable lipase from Pseudomonas species. Culture conditions and properties of the crude enzyme' Seite 267 ;Spalte R

## Beschreibung

Die vorliegende Erfindung betrifft die Verwendung spezieller Lipasen zur Herstellung von Arzneimitteln.

Lipasen spielen eine wichtige Rolle bei der Fettverdauung, da sie die Abspaltung von Fettsäuren aus den Fetten katalysieren. Lipasen werden daher in der Therapie zur Behandlung von Verdauungsstörungen eingesetzt, die auf Enzymmangel beruhen. Solche liegen beispielsweise bei Pankreasinsuffizienz, chronischer Pankreatitis und nach Magenresektion vor. Die hierbei verwendeten Lipasen sind zum großen Teil Produkte auf der Basis von entfettetem, getrocknetem und gemahlenem Schweinepankreas. Solche Schweinepankreaspräparationen weisen jedoch mehrere gravierende Nachteile auf:
1. Sie besitzen eine geringe spezifische Aktivität und müssen daher in einer Menge von bis zu 5 bis 10 g pro Tag eingesetzt werden.
2. Ihr Aktivitätsbereich liegt zwischen pH 5 und pH 9. Sie zeigen daher keine lipolytische Aktivität bei der Magenpassage.
3. Sie zeigen eine befriedigende Stabilität erst bei pH-Werten von 6 an aufwärts. Sie müssen daher entweder in säurefester Form oder in sehr hoher Dosis verabfolgt werden.
4. Die für pharmazeutische Zwecke hergestellten Lipasen liegen nicht in reiner Form vor. Sie enthalten Proteasen und Amylasen und sind damit bei bestimmten maldigestiven Krankheitsformen kontraindiziert.

Es wurde bereits vorgeschlagen, Lipasen zur Therapie der Maldigestion durch Züchtung von Pilzen der Gattung Aspergillus, Penicillium, Mucor, Candida oder Rhizopus herzustellen. In der DE-OS 16 42 654 und in der EP-A-0 387 945 werden die fermentative Herstellung und die Aufreinigung einer Lipase aus dem Pilz Rhizopus arrhizus zu therapeutischen Zwecken beschrieben.

Gegenstand der Erfindung ist die Verwendung von bakteriellen Lipasen, die eine positive immunologische Kreuzreaktion mit den Antikörpern der Lipase zeigen, die vom Mikroorganismus Pseudomonas spec. DSM 6483 gebildet wird, zur Herstellung von Arzneimitteln zur Therapie der Maldigestion. Zur Differenzierung der verwendbaren bakteriellen Lipasen benötigt man Antikörper gegen Lipase aus dem genannten Mikroorganismus. Die Lipasen lassen sich aus Bakterien gewinnen, indem man diese in einem Nährmedium züchtet und das Enzym aus der Kulturbrühe isoliert. Geeignet sind Nährmedien, die Kohlenstoffquellen, Stickstoffquellen, anorganische Salze und gegebenenfalls geringe Mengen an Spurenelementen und Vitaminen enthalten. Als Stickstoffquellen können anorganische oder organische Stickstoffverbindungen oder Materialien, die diese Verbindungen enthalten, verwendet werden. Beispiele sind: Ammoniumsalze, Nitrate, Maisquellwasser, Hefeautolysat, Hefeextrakt und hydrolisiertes Casein. Als Kohlenstoffquellen können Zucker wie Glucose, Polyole wie Glycerin oder auch organische Säuren wie Citronensäure oder Fettsäuren verwendet werden. Besonders geeignet als Kohlenstoffquelle sind pflanzliche Öle wie Sojaöl, Leinöl oder Olivenöl. Beispiele für die anorganischen Salze sind die Salze von Calcium, Magnesium, Mangan, Kalium, Zink, Kupfer, Eisen und anderen Metallen. Als Anionen der Salze sind besonders Phosphat-und Nitrationen zu nennen. Bevorzugte Züchtungstemperaturen liegen bei 25 bis 33°C. Der pH-Wert des Mediums wird bei 6 bis 7,5, vorzugsweise bei 6,5 bis 7 gehalten. Er wird durch 2n-Schwefelsäure oder Ammoniak eingestellt und während der Fermentation konstant gehalten. Die Kulturen werden als Submerskultur unter intensiver Belüftung und Rühren ausgeführt. Es wird bis zur Konstanz der Aktivität bei zwei aufeinanderfolgenden Enzymaktivitätsmessungen im Abstand von drei Stunden fermentiert. Im allgemeinen ist eine Inkubationsdauer von 40 bis 60 Stunden ausreichend.

Auf diese Weise lassen sich mit Bakterienstämmen, welche direkt aus natürlichen Habitaten isoliert wurden, Enzymausbeuten von 50 bis 500 mg pro l Kulturbrühe erzielen. Die Enzymausbeute läßt sich durch Mutagenese mit chemischen Agenzien oder UV-Licht mit einer nachgeschalteten Selektion auf verbesserte Lipaseproduktivität steigern.

Das Enzym wird aus der Kulturbrühe in üblicher Weise abgetrennt. Die Brühe wird zentrifugiert oder filtriert, um die Mikroorganismen und unlösliches Material abzutrennen. Sodann wird die flüssige Phase gesammelt, um die Lipase zu gewinnen. Dies geschieht durch Ausfällen mit einem mit Wasser mischbaren organischen Lösungsmittel (Alkohol oder Aceton z.B.) oder indem ein Salz wie Ammoniumsulfat zugesetzt wird. Zur Erhöhung der spezifischen Aktivität und zur weiteren Abreicherung von Verunreinigungen des in der beschriebenen Weise erhaltenen Rohproduktes ist es möglich, dieses wieder aufzulösen und erneut auszufällen, beispielsweise durch fraktioniertes Ausfällen durch Zusatz von Lösungsmitteln oder von Salz. Eine weitere Möglichkeit zur Reinigung des Rohproduktes stellen Querstromfiltrationen der das Enzym enthaltenden Lösung über geeignete Ultrafiltrationsmembranen dar, wobei niedermolekulare Verunreinigungen die Membran passieren, das Enzym aber zurückgehalten wird.

Zur Testung der so gewonnenen Lipasen auf ihre Brauchbarkeit benötigt man Antikörper gegen die Lipase, die sich aus Pseudomonas spec. DSM 6483 wie oben beschrieben gewinnen läßt. Hierzu wird diese Lipase in Zeitabständen von 10 bis 20 Tagen so lange Kaninchen injiziert, bis deren Seren einen genügend hohen Antikörpertiter zeigen. Zur Testung der Lipasen kann das so erhältliche Serum direkt in einem ELISA verwendet werden.

Bakterielle Lipasen, die eine positive immunologische Kreuzreaktion mit den oben genannten Lipasen zeigen, sind beispielsweise die Lipase aus Pseudomonas fluorescens (erhältlich von Enzymatix Ltd., Cambridge, UK) und die Lipase aus Pseudomonas spec. DSM 6535.

Die bakteriellen Lipasen zeigen einen Aktivitätsbereich von pH 3 bis pH 9. Darüberhinaus läßt sich für die erfindungsgemäß zu verwendenden bakteriellen Lipasen eine deutlich geringere Hemmwirkung durch Desoxycholsäure im Vergleich zur pilzlichen Lipase aus Rhizopus arrhizus nachweisen. Dies bedeutet, daß sich die lipolytische Aktivität der erfindungsgemäß zu verwendenden bakteriellen Lipasen mit größerer Effizienz als die marktgängiger Produkte zur Therapie der Maldigestion im Gastrointestinaltrakt nutzen läßt.

Die nach dem obigen Verfahren gewonnenen Produkte haben weiter den Vorteil, daß in ihnen die Lipase-Konzentration sehr hoch ist, so daß man nur geringe Substanzmengen (etwa 0,2 g) applizieren muß. Dies stellt eine drastische Verbesserung sowohl gegenüber Pankreatin als auch gegenüber der Pilzlipase dar.

Als weiterer Vorteil der bakteriellen Enzyme läßt sich anführen, daß sich die beschriebenen Präzipitate als Monopräparate ohne begleitende proteolytische bzw. amylolytische Enzymaktivitäten herstellen lassen. Dies bedeutet insofern einen Vorteil, als aufgrund der Anwesenheit von Proteasen und Amylasen Lipase in Pankreatins nicht in jedem Fall therapeutisch anwendbar sind: Bei Kindern mit Mukoviszidose ist der Anteil der Amylasen nicht wünschenswert, bei Patienten mit akuter Pankreatitis bzw. aktiven Schüben einer chronischen Pankreatitis sind Lipasen therapeutisch wünschenswert, Proteasen aber kontraindiziert.

Diese Lipasen eignen sich sehr gut zur Therapie von Maldigestionen unterschiedlicher Genese, d.h. ungenügender Verdauung des Darminhalts infolge Enzymmangels (z.B. nach Magenresektion, bei Pankreasinsuffizienz, Leberkrankheiten und bzw. Gallemangel). Die Lipasen werden oral in Form von Tabletten, Dragees und anderen festen Darreichungsformen verabfolgt. Der Gehalt der einzelnen Applikationsformen liegt vorzugsweise zwischen 10.000 und 100.000 Enzymeinheiten nach F.I.P. (= Monographie "Pankreas-Pulver" DAB9, Seite 1127/Ph.Eur., 2nd Edition, Serial No. 350). Pro Patient und Tag werden zwischen 20.000 und 400.000 Enzymeinheiten verabfolgt.

Die Lipasen können in den gebräuchlichen galenischen Applikationsformen fest oder flüssig angewendet werden, z.B. als Tabletten, Filmtabletten, Kapseln, Pulver, Granulate, Dragees oder Lösungen. Diese werden in üblicher Weise hergestellt. Die Wirkstoffe können dabei mit den üblichen galenischen Hilfsmitteln wie Tablettenbindern, Füllstoffen, Konservierungsmitteln, Tablettensprengmitteln, Fließreguliermitteln, Weichmachern, Netzmitteln, Dispergiermitteln, Emulgatoren, Lösungsmitteln, Retardierungsmitteln, Antioxidantien und/oder Treibgasen verarbeitet werden (vgl. H. Sucker et al. Pharmazeutische Technologie, Thieme-Verlag, Stuttgart, 1978). Die so erhaltenen Applikationsformen enthalten den Wirkstoff normalerweise in einer Menge von 10 bis 90 Gew.-%.

### Beispiel 1

a) Herstellung und Reinigung der Lipase aus Pseudomonas spec. DSM 6483
Zur Züchtung des Mikroorganismus Pseudomonas spec. DSM 6483 wurde folgendes Medium verwendet:

| | g/l |
|---|---|
| KH₂PO₄ | 20 |
| Na₂HPO₄ | 10 |
| MgSO₄ | 5 |
| CaCl₂ x 2H₂O | 3 |
| FeSO₄ x 7H₂O | 0,5 |
| MnSO₄ x 4H₂O | 0,005 |
| CoCl₂ x 6H₂O | 0,005 |
| CuSO₄ x 5H₂O | 0,005 |
| ZnSO₄ x 7H₂O | 0,005 |
| Hefeextrakt | 5 |

Als Kohlenstoffquelle diente raffiniertes Sojaöl, welches mit einer konstanten Zudosierrate von 1 g/l x h zugepumpt wurde. Der pH-Wert wurde über die gesamte Fermentationszeit mit Hilfe von 2 n H₂SO₄ und 25 %igem NH₄OH konstant bei pH 6,5 gehalten.
Die Impfkultur wurde durch Impfen von 400 ml Nutrient-broth-Medium pH 6,5 mit dem Mikroorganismrns Pseudomonos spec. DSM 6483 erhalten.
Die Impfkultur wurde 10 h bei 30°C auf einer Schüttelvorrichtung inkubiert.
Das Medium wurde bei 30°C und einem pH-Wert von 6,5 mit 5 Volumenteilen der Impfkultur pro 100 Volumenteile Medium beimpft. Die Hauptzüchtung wurde bei 30°C in 10 l-Rührfermentern mit 8 l Inhalt durchgeführt. Die Rührgeschwindigkeit der eingebauten Blattrührer betrug 1000 Umdrehungen pro Minute, die Belüftungsrate lag bei einem Volumen Luft pro Minute und Volumen Fermentationsbrühe. Nach 60 h zeigte die Fermentationsbrühe bei zwei aufeinanderfolgenden Aktivitätsmessungen eine konstante Aktivität von 300 Enzymeinheiten nach F.I.P.. Daraufhin wurde die Fermentation abgebrochen und die gebildete Lipase folgendermaßen aus der Fermentationsbrühe isoliert:
Der Fermenteraustrag wurde mit n-Propanol auf einen Volumenanteil von 65 % Alkohol verdünnt. Biomasse und ausgefällte Nebenprodukte wurden durch Zentrifugation abgetrennt. Die klare, alkoholische Enzymlösung wurde unter Vakuum auf ein Drittel des Ausgangsvolumens eingeengt. Das so erhaltene Enzymkonzentrat wurde im Diafiltrationsmodus (Cellulosetriacetat-Querstromfiltrationsmodule, Trenngrenze 20.000 nominelles Mol.-Gewicht, Fa. Sartorius, Göttingen) mit drei Volumina H₂O gewaschen und danach per Filtration auf ein Viertel des Ausgangsvolumens eingeengt. Aus diesem wäßrigen Enzymkonzentrat wurde Lipase ausgefällt, indem n-Propanol bis zu einem Volumenanteil von 85 % zugegeben wurde. Der die Lipaseaktivität enthaltende Niederschlag wurde, durch Zentrifugation geerntet und in einer 65 Volumenteile n-Propanol enthaltendem, wäßrigen Lösung aufgenommen. Das Gewichtsverhältnis Niederschlag zu n-Propanol/Wasser-Gemisch lag bei 1 zu 10.
Durch Zentrifugation wurde ungelöster Niederschlag abgetrennt. Aus dem klaren Überstand der Zentrifugation wurde die Lipase ausgefällt, indem der n-Propanolanteil auf 80 Volumenanteile erhöht wurde. Der Niederschlag wurde durch Zentrifugation geerntet und gefriergetrocknet. Das so erhaltene Enzympulver zeigte eine spezifische Aktivität von 7100 Enzymeinheiten nach F.I.P. pro Milligramm Protein.
b) In analoger Weise lassen sich auch Lipasen aus anderen Pseudomonadaceen herstellen und isolieren.

Eine sehr gute Lipase läßt sich aus Pseudomonas spec. DSM 6535 gewinnen.

Die taxonomische Untersuchung des Stammes DSM 6535 ergab folgende Eigenschaften:
Zellmorphologie: Durchmesser von 0,8 bis 1,0 µm bei einer Länge von 1,5 bis 2,0 µm
Gramfärbung: negativ in allen Wachstumsphasen
Sporen: keine
Beweglichkeit: vorhanden
Kein Wachstum bei 45°C bzw. 41°C
Wachstum bei 37°C
Wachstumsoptimum bei 30°C
Katalase: positiv
Oxidase: schwach positiv
keine fermentative Verwertung von Glucose
Strikt aerobes Wachstum
Die aufgelisteten Eigenschaften lassen eine Zuordnung von DSM 6535 zur Gattung Pseudomonas zu.

Durch die Identifizierung folgender physiologischer Merkmale sollte die Artzuordnung ermöglicht werden:
Arginindihydrolase: vorhanden
Lysindecarboxylase: nicht vorhanden
Ornithindecarboxylase: nicht vorhanden
Pigmentbildung auf King B-Medium: negativ
Hydrolyse von Tween ® 80: positiv
Hydrolyse von Casein: schwach positiv
Hydrolyse von Gelatine: schwach positiv
Hydrolyse von Stärke: positiv
Hydrolyse von Harnstoff: positiv
Hydrolyse von Aesculin: schwach positiv
Reduktion von Nitrat zu Nitrit: positiv
Levanbildung aus Saccharose: negativ
Lecithinase: negativ
β-Galactosidase: positiv
Verwertung von
   - Glucose:: positiv
   - Mannose:: positiv
   - Mannit:: positiv
   - Inosit:: positiv
   - N-Acetylglucosamin:: schwach positiv
   - Gluconsäure:: positiv
   - Citronensäure:: positiv
   - Äpfelsäure:: negativ
   - Phenylessigsäure:: negativ
   - Benzylamin:: positiv
   - Trehalose:: positiv

Aufgrund der physiologischen Eigenschaften von DSM 6535 ist keine eindeutige Artzuordnung möglich.

Daher wurde eine weitergehende Einordnung über die Fettsäurezusammensetzung der Zellwand versucht:
Das Vorkommen folgender Fettsäuren erlaubt eine zweifelsfreie Zuordnung von DSM 6535 zur rRNA-Homologiegruppe 2 der Pseudomonaden: 14:0 3-OH, 16:0 2-OH, 16:0 3-OH, 16:1 2-OH und 18:1 2-OH.

Ein Vergleich des Fettsäuremusters mit denen pflanzenpathogener Bakterien zeigt eine Ähnlichkeit mit dem Pseudomonas cepacia/gladioli-Komplex innerhalb der rRNA-Homologiegruppe 2.

Herausragendes physiologisches Merkmal von DSM 6535 ist jedoch die Lipaseproduktivität:
Unter den oben genannten Fermentationsbedingungen können mit DSM 6535 bis zu 3,2 g Lipase pro Liter Fermentationsmedium hergestellt werden.

### Beispiel 2

### Herstellung der Antikörper und Durchführung des Elisa

Gleiche Volumina einer Lösung von 0,1 mg/ml Antigen (= Lipase aus Pseudomonas spec. DSM 6483) und von Freudschem Adjuvans wurden gemischt, bis eine homogene Emulsion entstand. Zwei weiblichen Kaninchen wurden nach folgendem Zeitplan 2 ml-Proben dieser Emulsion injiziert:

Am Tag 0 wurde Antigen in komplettem Freudschen Adjuvans gegeben. Danach wurde in vierzehntägigem Abstand zweimal Antigen in inkomplettem Freudschem Adjuvans und danach Antigen ohne Adjuvans gegeben, bis ein Antikörpertiter erreicht war, der genügend Antikörper für den im folgenden beschriebenen Elisa-Test lieferte. Der Titer des Anti-Pseudomonas spec. DSM 6483-Serums wurde im Elisa nach folgender Vorgehensweise bestimmt:
Schritt (1)
   Mikrotiterplatten wurden mit Antigen beschichtet, wobei das Antigen in einer Konzentration von 1 bis 10 µg/ml in 0,05 M NaHCO₃ pH 9,2 gelöst wurde.
Schritt (2)
   Überschüssige Bindungsstellen wurden mit 1 % Rinderserumalbumin in phosphatgepufferter Saline (PBS) abgesättigt.
Schritt (3)
   Mikrotitergefäße wurden 3 mal mit 0,05 % Tween® 20 in PBS gewaschen.
Schritt (4)
   11 Verdünnungen des Kaninchenantiserums in PBS mit 0,5 % Tween® 20 (in 2er Schritten) wurden aufgebracht.
Schritt (5)
   Waschen wie Schritt 3.
Schritt (6)
   Biotinylierter anti-Kaninchen-IgG-Antikörper 1:10000, verdünnt in 0,1 % Rinderserumalbumin in PBS, wurde aufgebracht.
Schritt (7)
   Waschen wie Schritt 3.
Schritt (8)
   Es wurde Streptavidin-Peroxidase-Komplex mit dem Antikörper-Immunglobulin-Komplex zur Reaktion gebracht (Verdünnung 1:10000).
Schritt (9)
   Waschen wie Schritt 3.
Schritt (10):
   Als Peroxidasesubstratlösung wurde 0,42 mM Tetramethylbenzidin in 0,1 M Na-acetat pH 4,9, enthaltend 14,7 µl 3 % H₂O₂ eingesetzt.
Schritt (11)
   Die Enzymreaktion wurde mit 2 M H₂SO₄ gestoppt.

Der Titer des anti-Pseudomonas spec. 6483 wurde durch Messung der Absorption bei 450 nm bestimmt.

Alle Lipasen, welche eine positive immunologische Kreuzreaktion mit den Pseudomonas spec. DSM 6483-Antikörpern zeigen, die, wie oben beschrieben, hergestellt wurden, sind Lipasen gemäß vorliegender Erfindung.

Typische Beispiele für solche Lipasen sind:
Lipase aus Pseudomonas fluorescens (Handelsprodukt der Fa. Enzymatix Ltd., Cambridge UK) und Lipase aus Pseudomonas spec. DSM 6535.

### Eigenschaften der Lipasen

### Beispiel 3

Spezifische Aktivitäten verschiedener Lipasepräparationen mikrobiellen Ursprungs im Vergleich mit Pankreatin.

Die Aktivitätsbestimmungen wurden nach zwei unterschiedlichen Bestimmungsmethoden durchgeführt:
1) Lipase-Aktivitätsbestimmung für Pankreas-Pulver (Monographie "Pankreas-Pulver" DAB 9, Seite 1127/Ph.Eur., 2nd Edition, Serial Nr. 350) Enzymtest bei 37°C, pH 9, 10 mM Taurocholat.
2) Lipase-Aktivitätsbestimmung für mikrobielle Lipasen (Pharmaceutical Enzymes, Herausgeber: R. Ruyssen, A. Lauwers, E. Story-Scientia P.V.B.A. 1978, S. 210-213), Enzymtest bei 37°C, pH 7, 0,5 mM Taurocholat.

Für die Proteinbestimmung wurde ein Testkit der Firma Bio-Rad Laboratories GmbH verwendet. Dieser Testkit basiert auf der Methode von M. Bradford (Anal. Biochem. (72), 248 (1976)).

Wie die nachfolgende Tabelle I zeigt, wurden für die hier beschriebenen bakteriellen Lipasen mit der für Pankreatin vorgeschriebenen Methode des Deutschen Arzneibuches deutlich höhere spezifische Aktivitäten gemessen als bei pilzlichen Lipasen oder bei Pankreatin. Übereinstimmung zwischen Pankreatin und den bakteriellen Lipasen besteht insofern, als bei beiden Enzymen die bei pH 9 gemessenen spezifischen Aktivitäten über den bei pH 7 - im Test für mikrobielle Lipasen - gemessenen Aktivitäten liegen.

Die untersuchten pilzlichen Lipasen verhalten sich in diesem Punkt umgekehrt. Daher sind ihre maximalen spezifischen Aktivitäten nicht direkt mit der des Pankreatin vergleichbar.

**Tabelle I**

| Lipase aus | Spezifische Aktivität pro Milligram Protein nach | |
|---|---|---|
| | Pharmaceutical Enzymes | F.I.P. |
| Pseudomonas spec. DSM 6483 (Beispiel 1) | 6696 | 7108 |
| Pseudomonas spec. DSM 6535 (Beispiel 1) | 5064 | 6290 |
| Pankreatin | 226 | 430 |
| Rhizopus arrhizus | 942 | 317 |
| Candida cylindracea | 4597 | 41,6 |
| Mucor miehei | 294 | 169 |
| Aspergillus niger | 394 | 0 |
| Penicillium roquefortii | 195 | 83,1 |
| Geotrichum candidum | 172 | 63,9 |

### Beispiel 4

### pH-Abhängigkeit bakterieller Lipasen im Vergleich zu Pankreatin

Um den Zusammenhang zwischen pH-Wert und lipolytischer Aktivität zu untersuchen, wurde ein modifiziertes Testsystem für mikrobielle und tierische Lipasen verwendet (Ch. Unterberg in: Fette, Seifen, Anstrichmittel; Band 88; 1986, S. 561-564). Die in Tabelle II aufgeführten Werte zeigen, daß die bakteriellen Lipasen im Vergleich zu Pankreatin einen weit in den sauren pH-Bereich ausgedehnten Aktivitätsbereich aufweisen. Damit weisen diese Lipasen den Vorteil auf, ihre Aktivität schon im Magen entfalten zu können.

**Tabelle II**

| pH-Wert | relative lypolitische Aktivität (%) | | |
|---|---|---|---|
| | Pankreatin | Pseudomonas spec. DSM 6483 | Pseudomonas spec. DSM 6535 |
| 3,0 | 0 | 0 | 14,3 |
| 3,5 | 0 | 14,3 | 27,4 |
| 4,0 | 0 | 16,9 | 20,7 |
| 4,5 | 5,0 | 29,9 | 34,1 |
| 5,0 | 20,0 | 52,0 | 34,1 |
| 5,5 | 27,6 | 52,0 | 35,4 |
| 6,0 | 30,8 | 52,0 | 37,8 |
| 6,5 | 46,6 | 58,4 | 46,3 |
| 7,0 | 61,7 | 71,4 | 56,1 |
| 7,5 | 76,7 | 79,2 | 76,8 |
| 8,0 | 86,7 | 100,0 | 100,0 |
| 8,5 | 90,0 | 84,4 | 68,3 |
| 9,0 | 100,0 | 100,0 | 69,5 |

### Beispiel 5

### Wirkung von Natriumdesoxycholat auf die Lipasen aus Pseudomonas spec. DSM 6535 sowie Rhizopus arrhizus

Um den Einfluß des Gallensalzes Natriumdesoxycholat auf die Aktivität mikrobieller Lipasen zu untersuchen, wurde die Aktivitätsbestimmung für Pankreas-Pulver (Monographie "Pankreas-Pulver" DAB 9, Seite 1127/Ph. Eur., 2nd Edition, Serial No. 350) verwendet. Anstelle von Taurocholat wurde Natriumdesoxycholat in Konzentrationen von 0 bis 10 mM im Enzymtest eingesetzt.

Wie die nachfolgende Tabelle III am Beispiel der Lipase aus Pseudomonas spec. DSM 6535 verdeutlicht, besitzen die gemäß vorliegender Erfindung zu verwendenden bakteriellen Lipasen gegenüber einer zum Vergleich herangezogenen pilzlichen Lipase aus Rhizopus arrhizus den Vorteil, daß sie insbesondere bei Natriumdesoxycholat-Konzentrationen von über 5 mM nicht gehemmt werden, wohingegen das pilzliche Enzym schon deutliche Inaktivierung zeigt.

**Tabelle III**

| Konzentration von Na-desoxycholat (mM) | relative lipolytische Aktivität (%) | |
|---|---|---|
| | Pseudomonas spec. DSM 6535 | Rhizopus arrhizus |
| 0 | 100 | 100 |
| 0,5 | 114 | 180 |
| 1 | 144 | 160 |
| 2,5 | 177 | 118 |
| 3,5 | 100 | 100 |
| 5 | 60 | 69 |
| 6,5 | 115 | 64 |
| 7,5 | 117 | 50 |
| 10 | 120 | 0 |

## Patentansprüche

1. Verwendung von bakteriellen Lipasen, die eine positive immunologische Kreuzreaktion mit den Antikörpern der Lipase zeigen, die vom Mikroorganismus Pseudomonas spec. DSM 6483 gebildet wird, zur Herstellung von Arzneimitteln zur Therapie der Maldigestion.

2. Verwendung der bakteriellen Lipase aus Pseudomonas spec. DSM 6535 zur Herstellung von Arzneimitteln zur Therapie der Maldigestion.

3. Pseudomonas spec. DSM 6535

## Claims

1. The use of bacterial lipases which show a positive immunological cross-reaction with the antibodies to the lipase produced by the microorganism Pseudomonas spec. DSM 6483 for producing drugs for the therapy of maldigestion.

2. The use of the bacterial lipase from Pseudomonas spec. DSM 6535 for producing drugs for the therapy of maldigestion.

3. Pseudomonas spec. DSM 6535.

## Revendications

1. Utilisation de lipases bactériennes ayant une réaction immunologique croisée positive avec les anticorps de la lipase formée par le microorganisme Pseudomonas spec. DSM 6483, pour la préparation de médicaments servant à la thérapie des troubles de la digestion.

2. Utilisation de la lipase bactérienne de Pseudomonas spec. DSM 6535 pour la préparation de médicaments prévus pour la thérapie des troubles de la digestion.

3. Pseudomonas spec. DSM 6535.
